# FASCICULE DE BREVET EUROPEEN

(11) **EP 1 287 112 B1**
(45) Date de publication et mention de la délivrance du brevet: **01.09.2004**
(21) Numéro de dépôt: 01945386.9
(22) Date de dépôt: 08.06.2001
(51) Int. Cl.: C12M 1/12, B01D 63/02

(54) **PREPARATION D'UN ECHANTILLON A ANALYSER A PARTIR D'UN ECHANTILLON DE TRES GRAND VOLUME**
VERFAHREN ZUR VOBEREITUNG EINER PROBE ZUR ANALYSE AUSGEHEND VON EINER PROBE MIT SEHR GROSSEM VOLUMEN
PREPARING A SAMPLE TO BE ANALYSED FROM A SAMPLE WITH VERY LARGE VOLUME

(30) Priorité: 09.06.2000 FR 0007459
(43) Date de publication de la demande: 05.03.2003
(73) Titulaire: BIO MERIEUX, 69280 Marcy l'Etoile (FR)
(72) Inventeur: GUILLOT, Emmanuelle, F-78100 Saint Germain en Laye (FR); DURAND-BOURLIER, Laurence, F-92140 Clamart (FR); BULTEAU, Stéphane, F-69007 Lyon (FR)
(74) Mandataire: Guerre, Dominique
(86) Numéro de dépôt international: PCT/FR2001/001786
(87) Numéro de publication internationale: WO 2001/094527

(56) Documents cités:
- GB-A- 2 339 155

## Description

L'invention concerne le domaine de l'analyse des contaminants présents dans les milieux liquides, qui nécessite le traitement d'échantillons de volume important comportant à l'état dilué une multiplicité de microorganismes de tailles différentes (virus, bactéries, parasites) ainsi que diverses macromolécules organiques ou minérales, pour obtenir un échantillon liquide final de faible volume, comportant à l'état concentré pratiquement la même composition relative en contaminants.

Cet enrichissement en contaminants, notamment en microorganismes, et l'exigence de faibles volumes à analyser, sont rendus nécessaires par l'utilisation de techniques d'analyse biochimique ou biologique, notamment les immunoessais et les techniques de biologie moléculaire, par exemple pour la multidétection.

On connaît des moyens de séparation et de concentration des microorganismes, notamment des virus, par exemple par adsorption. Outre le fait que ces moyens ne garantissent pas la viabilité des microorganismes, ils ne permettent pas d'obtenir des échantillons de volume compatible avec les techniques d'analyse précédemment citées.

D'autres moyens mis en oeuvre pour la récupération de microorganismes dans les milieux liquides font appel à des procédés de filtration et à l'analyse d'un seul type de microorganisme mais ne permettent pas toujours d'atteindre des résultats quantitatifs.

On notera par exemple pour les bactéries et les parasites, l'utilisation, selon les recommandations de méthodes normées, de membranes planes avec des pores de taille comprise entre 0,2 µm et 1 µm en fonction de l'espèce.

On connaît notamment de FR-B-2693474, une technique basée sur l'ultrafiltration tangentielle mettant en oeuvre de façon classique une ou plusieurs membranes et permettant la séparation de microorganismes de tailles différentes en garantissant la viabilité desdits microorganismes.

Cependant l'exigence sur le volume de l'échantillon liquide final ne permet pas son utilisation pour préparer des échantillons à analyser par des techniques d'analyse biochimique ou biologique.

On connaît de GB2339155, l'utilisation d'un moyen de filtration dans lequel sont disposées parallèlement les unes aux autres une multiplicité de fibres, ce moyen étant utilisé en voie interne sans aucune étape complémentaire de rétrolavage et ne permettant d'obtenir qu'un très faible rendement, de l'ordre de 28 % en comparaison de l'utilisation d'un moyen de filtration dans lequel les fibres sont disposées en U, ce moyen étant lui utilisé en voie externe, mais cette utilisation ne permettant cependant pas d'obtenir les résultats attendus.

On connaît également de Otaki et col. (Wat. Sci. Tech. 37, 10, 107-116, 1998) pour valider les performances de membranes de microfiltration, l'utilisation en continu et en mode tangentiel d'un dispositif d'ultrafiltration sur fibres creuses, avec analyse de prélèvements effectués périodiquement par prélèvement sur l'alimentation, le filtrat et le concentrat respectivement.

L'utilisation de moyens de filtration et d'ultrafiltration en mode tangentiel implique la présence d'un circuit permettant la recircutation de tout ou partie du concentrat pour maintenir une vitesse tangentielle à la membrane permettant la filtration des particules en suspension tout en évitant le colmatage rapide des membranes utilisées ("Microfiltration and Ultrafiltration - Principles and Applications", Leos J. Zeman, Andrew L. Zydney, Marcel Dekker, Inc. New York - Basel - Hong Kong 1996).

La recirculation des fluides soumis à la filtration induit des durées de filtration allongées, et surtout des volumes supplémentaires dans lesquels une partie du concentrat et des microorganismes ne pourront pas être récupérés sans incidence sur les volumes finaux.

Les volumes de concentrat importants, dus au volume des tuyauteries et également au volume d'éluant nécessaire pour extraire les microorganismes pouvant être restés sur les parois, et l'allongement des temps de filtration rendent ces techniques incompatibles avec les moyens d'analyse nécessitant de faibles volumes à analyser et, permettant d'apporter des résultats rapidement, par exemple pour répondre à des interrogations sur des contaminations d'eau potable ou tout autre fluide destiné à la consommation humaine.

L'invention a pour objet l'utilisation d'un moyen de filtration permettant d'obtenir dans un temps limité, à partir d'un échantillon liquide de départ de volume important et prédéterminé, un échantillon à analyser d'un volume suffisamment faible pour que les techniques d'analyse biochimique ou biologique, notamment les immunoessais et les techniques de biologie moléculaire, par exemple pour la multidétection, puissent être ensuite mises en oeuvre en garantissant la viabilité des microorganismes.

Le moyen de filtration utilisé selon l'invention est basé sur la technique de l'ultrafiltration sur fibres creuses en mode frontal.

Par mode frontal, par opposition au mode tangentiel, on entend tout passage en une passe de l'échantillon liquide de départ dans le moyen de filtration, sans recyclage d'au moins une partie du même échantillon à l'entrée dudit moyen de filtration.

L'utilisation de ce moyen d'ultrafiltration en mode frontal permet d'obtenir des concentrats de volume faible, d'effectuer la concentration d'un prélèvement dans une échelle de temps de l'ordre de l'heure au plus en un seul passage, tout en garantissant la viabilité des microorganismes, la multirécupération et des rendements de l'ordre de 100 %.

Par multirécupération, on entend la possibilité de récupérer dans l'échantillon final pratiquement tous les différents genres ou espèces de microorganismes présents dans l'échantillon de départ.

Ces rendements élevés sont obtenus en raison de l'inexistence de volumes, dits volumes morts, dus par exemple sur d'autres dispositifs à la présence de tuyauteries annexes, par exemple de recyclage, et par la fiabilité de la porosité sur toute la longueur de la fibre creuse.

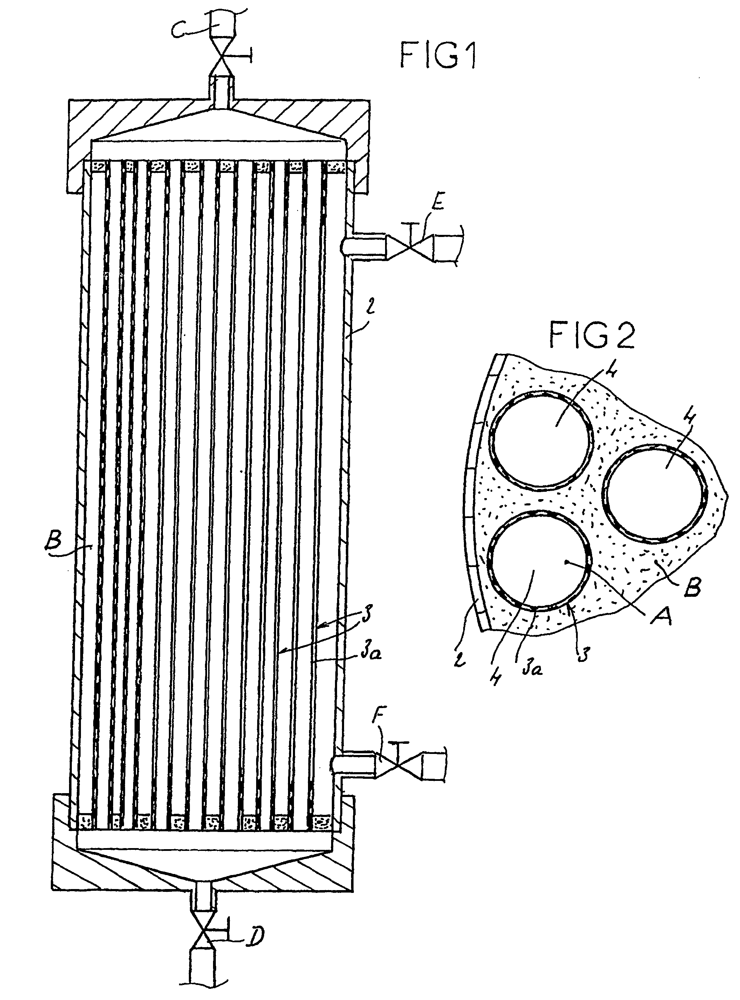

Les caractéristiques du moyen de filtration utilisé apparaîtront à la lecture de la description faite en référence au dessin annexé, dans lequel la Figure 1 représente de manière schématique et en coupe longitudinale un moyen de filtration selon l'invention, et la Figure 2 une vue en coupe transversale, à échelle agrandie, du même moyen de filtration. La taille des fibres a été volontairement exagérée, pour la compréhension de l'invention.

Le moyen de filtration comporte un carter (2) dans lequel sont disposées parallèlement les unes aux autres une multiplicité de fibres creuses (3), dont la paroi poreuse (3a) de chacune détermine une lumière (4), en sorte que ledit moyen comporte une voie interne (A) constituée par la multiplicité des lumières (4) en parallèle desdites fibres creuses respectivement, et une voie externe (B) correspondant au volume disponible entre les fibres et le carter.

Selon l'invention, ce moyen est utilisé pour le traitement d'un échantillon liquide de départ de volume important, comportant à l'état dilué une multiplicité de microorganismes de tailles différentes, pour obtenir un échantillon liquide final de faible volume, comportant à l'état concentré la même composition en microorganismes.

Lors de l'utilisation de ce moyen on effectue au moins une étape de concentration, pendant laquelle :
a) on dispose d'un seul moyen de filtration
b) ledit moyen de filtration est utilisé en mode frontal,
c) le seuil de coupure de la paroi poreuse (3a) des fibres est déterminé en sorte d'arrêter le micro-organisme de l'échantillon de départ de plus petite taille, et de manière discontinue :
d) pendant une phase de filtration, on introduit la totalité de l'échantillon liquide de départ par l'une des voies (A, B) du moyen de filtration, on évacue un filtrat par l'autre (B, A) des voies du moyen de filtration, et on accumule un concentrat dans ladite voie d'introduction de l'échantillon liquide,
e) pendant une phase de récupération après avoir fait circuler la totalité de l'échantillon, on arrête la circulation de l'échantillon liquide de départ dans le moyen (1) de filtration de ladite voie à ladite autre voie et on obtient l'échantillon liquide de sortie à partir du concentrat.

L'échantillon liquide de sortie constitué par le concentrat peut être récupéré soit par simple gravité, soit par utilisation d'un gaz comprimé, soit par agitation physique dudit moyen de filtration, mais également par élution avec toute substance adéquate, par exemple pour des microorganismes avec une solution saline type PBS (ex: 120 mmol NaCl, 2,7 mmol KCI, 10 mmol NaH2PO4) contenant éventuellement un détergent type TWEEN 20 et/ou un surfactant type BSA ("bovin serum albumin").

Selon un autre mode de réalisation, l'échantillon liquide de sortie constitué par le concentrat est soumis à une deuxième étape de concentration et constitue, alors dans cette deuxième phase de concentration, l'échantillon liquide de départ.

Les parois des fibres creuses (3a) peuvent être constituées de tout matériau permettant la fabrication de fibres creuses de porosité définie, tel que des membranes organiques, par exemple en acétate de cellulose, éthylcellulose, polyether sulfone, ou polyacrylonitrile, ou des membranes minérales, par exemple une céramique.

Le seuil de coupure de la paroi poreuse (3a) des fibres est déterminé en sorte d'arrêter le micro-organisme de l'échantillon de départ de plus petite taille. On utilisera donc des fibres de porosité de 10 à 100 nm, pour le domaine de l'ultrafiltration, pour que le concentrat comprenne tous les microorganismes de taille supérieure à celle des virus, ceux-ci étant compris, mais si la taille des particules recherchées l'exige, notamment pour des particules inférieures à 10 nm, des fibres de porosité de 1 à 10 nm pour le domaine de la nanofiltration peuvent être utilisées.

Selon un mode d'exécution, la voie d'introduction de l'échantillon de départ est la voie interne (A) et la voie d'extraction de l'échantillon de sortie est la voie externe (B), de sorte que les microorganismes s'accumulent à l'intérieur des fibres pour former le concentrat, tandis que le filtrat passe à l'extérieur des fibres dans le volume disponible entre les fibres et le carter (B).

L'échantillon à traiter constitué d'un prélèvement d'eau d'un volume compris par exemple de 10 à 100 litres, est introduit par une vanne (C) à l'intérieur des fibres creuses du module, entraîné par exemple par une aspiration exercée par une pompe péristaltique ou tout autre moyen, tel qu'une dépression créée du côté du filtrat par la vanne (E), les vannes (D) et (F) restant fermées.

Lorsque la totalité du volume à traiter est passée à travers le module, la pompe est arrêtée, la vanne (C) est fermée et déconnectée de la source d'échantillon.

Le carter est ensuite vidé pour évacuer le filtrat par exemple par la vanne (F) en mettant le module à la pression atmosphérique, par exemple en ouvrant la vanne (E) , puis en le mettant sous pression, par exemple sous 0,5 bar d'air comprimé, la vanne E étant alors refermée.

Le concentrat est ensuite recueilli par la vanne (D) en ouvrant la vanne (C), d'abord par gravité puis en terminant par la fermeture de la vanne (C) et l'injection d'air comprimé à l'intérieur des fibres.

Selon un autre mode d'utilisation, la voie d'introduction de l'échantillon de départ est la voie externe (B), et la voie d'extraction de l'échantillon de sortie est la voie interne (A), de sorte que les microorganismes s'accumulent à l'extérieur des fibres dans le volume disponible entre les fibres et le carter pour former le concentrat, et que le filtrat passe à l'intérieur des fibres pour être évacué.

L'échantillon à traiter constitué d'un prélèvement d'eau d'un volume compris par exemple de 10 à 100 litres est introduit par la vanne (E) à l'intérieur du carter, entraîné par exemple par une dépression, pouvant être créée par une aspiration exercée par une pompe péristaltique ou tout autre moyen, dépression créée du côté du filtrat à l'intérieur des fibres creuses par exemple par la vanne (D), la vanne (C) restant fermée.

Lorsque la totalité du volume à traiter est passé à travers le module, la pompe est maintenue en fonctionnement, la vanne (E) étant déconnectée de la source d'échantillon pour réduire le volume du concentrat contenu dans le carter du module ; cette réduction peut être totale ou partielle.

Lorsque le niveau de concentration du concentrat souhaité est atteint, la pompe est arrêtée et la vanne (D) est fermée ainsi que toutes les vannes.

Une solution d'éluant est éventuellement introduite dans le carter, puis le module est agité et le concentrat ou l'éluat est ensuite recueilli par la vanne (F) en terminant éventuellement par l'injection d'air comprimé par la vanne (E).

Les fibres, pour limiter les phénomènes d'adsorption peuvent avoir été préalablement traitées par exemple avec un détergent de type TWEEN et/ou un surfactant de type BSA ("bovin serum albumin").

Selon un mode d'exécution, la phase de filtration (C) est effectuée sans préfiltration de l'échantillon liquide de départ, si la teneur en matière en suspension des fluides à analyser le permet.

Pour le mode d'introduction par la voie interne (A), le volume du concentrat obtenu est directement lié au dimensionnement du moyen de filtration; pour le mode d'introduction par la voie externe (B) le volume dudit concentrat ne dépend pas du dimensionnement du moyen de filtration mais du volume laissé dans le carter et/ou du volume d'éluant introduit dans ce carter.

Le moyen de filtration sera dimensionné pour que le rapport entre le volume de l'échantillon de départ et le volume du concentrat obtenu soit au moins égal à 50 et préférentiellement compris entre 150 et 500, avec un temps de filtration compris entre 20 minutes et une heure.

Si l'application le permet, le module peut être réutilisé après avoir été soumis à un rétrolavage ou à un traitement avec un décontaminant par exemple une solution de chlore à 50 ppm associée ou non à un traitement enzymatique.

Deux modes de rétrolavage peuvent être mis en oeuvre selon que la voie d'introduction de l'échantillon est la voie interne (A) ou la voie externe (B), l'eau de lavage étant introduite à contre-courant de l'introduction de l'échantillon à analyser.

Lorsque la voie d'introduction de l'échantillon est la voie interne (A), par la vanne (C), le rétrolavage est effectué par introduction d'eau du réseau par la vanne (F) grâce à un moyen de refoulement, comme une pompe péristaltique placée entre la source d'eau et ladite vanne, la vanne (D) étant fermée et la vanne (E) restant momentanément ouverte.

Lorsque le carter est rempli, la vanne (E) est fermée et l'eau est contrainte de passer à l'intérieur des fibres pour sortir par la vanne (C) ou (D).

Lorsque la voie d'introduction de l'échantillon est la voie externe (B), le rétrolavage est effectué par introduction d'eau du réseau par la vanne (D), les vannes (E) et (F) étant fermées.

Lorsque les fibres sont remplies d'eau, c'est à dire lorsque l'on n'observe plus de présence de bulles d'air en sortie par la vanne (C), celle-ci est fermée et l'eau est contrainte de passer à l'extérieur des fibres dans le carter pour sortir par la vanne (E) ou la vanne (F).

L'usage unique du module peut être éventuellement prévu, ainsi que la stérilité initiale du module par stérilisation par traitement aux rayons gamma par exemple.

Dans un autre mode de réalisation, on peut utiliser un module sans joint, ce qui évite tout risque de contamination extérieure (fluide extérieur au module tel que l'air ambiant); cela évite aussi tout passage à travers les joints de contaminants du concentrat vers le filtrat. On réalise l'étanchéité en noyant à leurs deux extrémités respectivement les fibres creuses dans une résine, comme représenté dans la figure 1 et on prévient ainsi toute fuite de contaminant ce qui n'est pas le cas des systèmes comportant des joints.

Dans un autre mode de réalisation, pour obtenir un volume de concentrat compatible avec les méthodes d'analyse mises en oeuvre, le concentrat obtenu peut être soumis à une seconde étape de concentration.

Dans cette seconde étape de concentration le moyen de filtration utilisé peut être de même configuration que celui utilisé dans la première étape mais de volume plus faible, calculé en fonction du rapport entre le volume de départ et le volume de concentrat souhaité.

Les volumes traités étant de l'ordre de la centaine de ml, des moyens connus comme les dispositifs de filtration faisant appel aux techniques de séparation par centrifugation tels que les dispositifs commercialisés par la société Millipore sous l'appellation Centricon-Plus 80 peuvent être utilisés.

Ces moyens de filtration sont dimensionnés et les traitements sont effectués pendant un temps déterminé, en sorte que le rapport entre le volume de l'échantillon de départ et le volume de l'échantillon final est au moins égal à 5 000 et préférentiellement compris entre 15 000 et 100 000.

L'utilisation du moyen de filtration selon l'invention présente donc les avantages de permettre la multirécupération avec un rendement proche de 100 % des microorganismes présents dans de grands volumes de liquide à analyser, de garantir la viabilité des microorganismes ainsi récupérés, d'effectuer cette multirécupération dans des temps de l'ordre d'une heure au plus, compatibles avec les exigences, par exemple d'une surveillance sanitaire en continu, et d'obtenir des volumes de concentrat à analyser qui permettent la mise en oeuvre de techniques d'analyse biochimique ou biologique, notamment les immunoessais et les techniques de biologie moléculaire.

De façon non limitative les exemples suivants permettront d'illustrer l'utilisation d'un moyen de filtration selon l'invention :

### EXEMPLE 1:

### Matériel et méthode :

Utilisation d'un moyen de filtration comportant 1 m² de surface filtrante en fibres creuses d'acétate de cellulose.

L'échantillon constitué de 50 litres d'eau de réseau dopés avec le bactériophage MS2 est introduit par la voie interne;

La récupération du concentrat est effectuée par gravité et sous pression;

L'analyse du concentrat est conduite par la technique des plages de lyse mesurant à la fois la viabilité et l'infectiosité du bactériophage , soit en quantitatif (après dilutions de 10 en 10 de l'échantillon), soit en qualitatif (après amplification biologique par infection d'une culture bactérienne sensible au MS2).

### Protocole :

Une sonication des inocula est effectuée avant dopage et prélèvement de l'eau du robinet en cuve afin de désagréger les particules virales.

Après addition de 20 mg/l de thiosulfate de sodium (TSS (Merck 106516)), soit 1 g dans 50 l, l'échantillon est dopé avec MS2.

Un cycle de filtration est effectué, puis le carter est vidé, fermé et mis sous pression d'air (0.5 b). Ensuite le concentrat est récupéré par gravité. De l'air comprimé (0.5 b) est ensuite injecté également dans les fibres pour récupérer les derniers millilitres de concentrat restés dans les fibres.

La numération est ensuite effectuée par la technique des plages de lyse avec souche sensible *Escherichia coli* (*E*.*coli*) Hfr, en soniquant préalablement les concentrats.

### Préparation des inocula :

| Dilution MS2 | volume dilution MS2 | EPS | titre final (PFU/ml) |
|---|---|---|---|
| Stock | - | - | 2.10⁸ |
| I10⁷ | 500 µl stock | 9.5 ml | 10⁷ |
| I10⁶ | 1 ml de I10⁷ | 9 ml | 10⁶ |
| I10⁵ | 1 ml de I10⁶ | 9 ml | 10⁵ |
| I10⁴ | 1 ml de I10⁵ | 9 ml | 10⁴ |
| I10³ | 1 ml de I10⁴ | 9 ml | 10³ |
| I100 | 1 ml de I10³ | 9 ml | 100 |
| I10 | 1 ml de I100 | 9 ml | 10 |
| I1 | 1 ml de I10 | 9 ml | 1 |
| EPS = Eau Peptonée Saline ; PFU = Plaque Forming Unit (unité virale infectieuse); I10^{x} = Inoculum contenant 10^{x} PFU par ml. | | | |

### Filtration :

50 litres d'eau du robinet neutralisée au TSS sont filtrés et, après vidange du carter, on récupère un concentrat qui constituera un Blanc = Blanc 1 (B1).

Après rétrolavage pour nettoyer la membrane 50 litres d'eau du robinet neutralisée au TSS sont filtrés, et après vidange du carter, on récupère un concentrat qui constituera un Blanc = Blanc 2 (B2).

Après rétrolavage pour nettoyer la membrane, 50 litres d'eau du robinet sont neutralisés avec le TSS.

Après 15 minutes d'attente on prélève 1 litre pour diluer l'inoculum destiné à la cuve.

Le dopage est effectué avec 1 ml de I10 (10 PFU) dilué dans 1 litre.

Après un cycle de filtration le concentrat est récupéré (C10);

Un rétrolavage est effectué.

Dans cet exemple, les rétrolavages sont réalisés avec au moins 20 litres d'eau.

Puis la même procédure est répétée en dopant avec 1 ml I10⁴ (10⁴ PFU), Un prélèvement de filtrat en cours de filtration donne l'échantillon (F). A la fin de la filtration on récupère le concentrat (C10⁴) ;

Le dopage d'une fraction de 100 ml de B1 avec 1 ml de I10⁴ (10⁴ PFU) donne l'échantillon témoin Blanc Dopé (BD10⁴);

### Analyse :

- titration quantitative de I100, I10, I1, C10⁴, BD10⁴.

Les aliquots de chaque échantillon pur ainsi que les inocula sont prélevés en tube à essai et soumis à une sonication avant de procéder aux dilutions.

Les particules infectieuses sont comptées par la technique des plages de lyse selon le protocole suivant :

2 ml d'un Top Agar mou (0,9%) en surfusion (44°C) sont mélangés à 1 ml de suspension virale et 0,3 ml de culture *E.coli* Hfr en phase exponentielle de croissance. Le tout est rapidement et doucement mélangé puis versé sur toute la surface d'une gélose nutritive dure en boite de Petri. Après incubation à 37°C les plages de lyse sont lues le lendemain.

### Résultats obtenus :

| Dilutions | numérations | | | | moyennes | écart-type | moyenne globale | remarque |
|---|---|---|---|---|---|---|---|---|
| I 100 | 61 | 78 | 74 | 75 | 72,0 | 6,5 | 74,5 | |
| I 10 | 9 | 7 | 17 | 8 | 10,3 | 4,0 | | |
| I 1 | 1 | 1 | 0 | 0 | 0,5 | 0,5 | | |
| C10⁴ pur | 14 | 13 | 15 | *6 | 14,0 | 0,8 | 13,9 | *6 écarté: lecture douteuse |
| C10⁴ 10⁻¹ | 1 | 3 | 1 | 0 | 1,3 | 1,1 | | |
| BD10⁴ 10⁻¹ | 61 | 81 | 60 | 78 | 70,0 | 9,6 | 78,2 | |
| BD10⁴ 10⁻² | 13 | 19 | 14 | 18 | 16,0 | 2,5 | | |
| C10⁴ 10⁻¹, par exemple, désigne la dilution 1/10 du concentrat obtenu à partir de l'échantillon dopé avec 10⁴ PFU. | | | | | | | | |

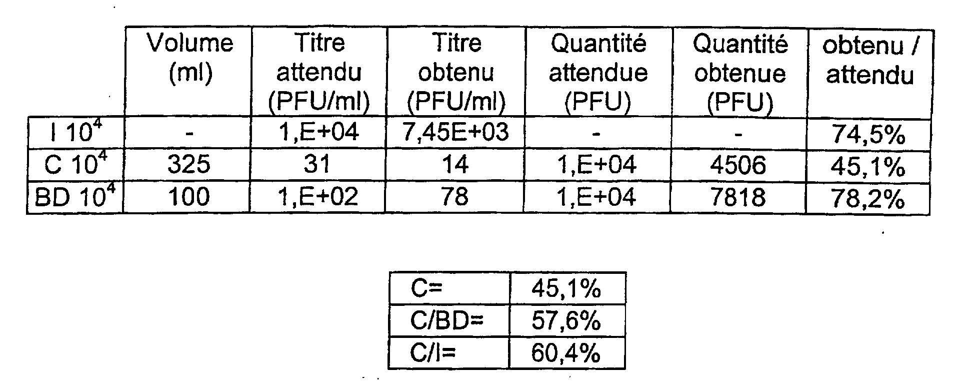

### Test qualitatif :

Détection de la présence ou de l'absence de phage sur I10⁷ (témoin positif = T+), F (1 prise de 50 ml), B2 (6x 50ml + reste pour couvrir tout l'échantillon), C 10 (6x 50 ml + reste pour couvrir tout l'échantillon).

Un volume d'échantillon est mélangé avec 1 volume de bouillon nutritif concentré deux fois. Après incubation 30 minutes à 37°C, on inocule avec une culture d'*E*.*coli* Hfr en phase exponentielle. Après incubation une nuit à 37°C (phase de multiplication virale), on étale une culture exponentielle d'E.coli Hfr sur une gélose nutritive en boite de Pétri et on laisse sécher. Des gouttes du bouillon d'amplification virale sont déposées et on laisse incuber à 37°C au moins six heures.

La présence de particules infectieuses est révélée par la présence de plages de lyse plus ou moins confluentes à l'endroit du dépôt de la goutte.

En cas d'absence de particules infectieuses, la croissance d'*E*.*coli* Hfr produit une couche cellulaire opaque à l'endroit du dépôt.

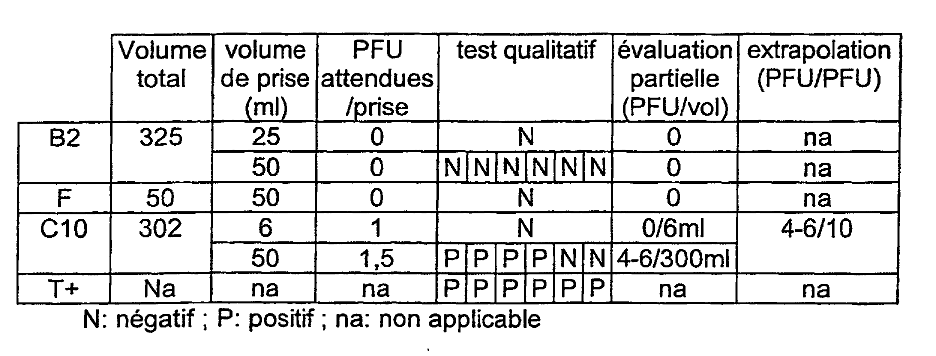

### Interprétation des résultats :

Le blanc (B2) ne contient pas de bactériophage infectieux: le module n'est donc pas préalablement contaminé par le MS2 et la concentration des 10 PFU qui est effectuée ensuite est valide;

Le prélèvement du filtrat (50 ml) effectué lors de la concentration des 10⁴ PFU ne contient pas de MS2 détectable: la membrane arrête donc efficacement ce virus de 25 nm de diamètre.

Quatre unités infectieuses ayant été détectées dans C10, le seuil de détection est donc inférieur. à 10 PFU.

Le rendement réel (c'est-à-dire rapporté au titre mesuré de l'inoculum: C/I) est de 60% pour un dopage de 10⁴ PFU en l'absence de procédé particulier de récupération (pas d'agitation ni d'élution), et en tenant compte de la viabilité car la mesure porte sur l'infectiosité du bactériophage.

Le rapport C/BD est très proche du rendement réel, ce qui montre qu'il n'y a pas d'interférence due au concentrat.

### EXEMPLE 2 :

### Matériel et méthode :

Utilisation d'un moyen de filtration comportant 0,6 m² de surface filtrante en fibres creuses d'acétate de cellulose

L'échantillon est constitué de 50 litres d'eau de réseau dopés avec des oocystes du parasite *Cryptosporidium parvum*. Il est introduit par la voie externe.

La récupération du concentrat est effectuée par agitation et collecte par gravité et sous pression;

L'analyse est faite par la technique de l'IMS-IFA: Immuno Magnetic Separation -Immuno Fluorescence Assay (voir méthode normée 1622, EPA-EPA-821-R-99-001 de l'Agence pour la Protection de l'Environnement aux U.S.A.) avec le kit Dynabeads anti-Cryptosporidium de Dynal, ref. 730.01 (respect de l'intégrité des oocystes).

### Protocole :

Après prélèvement de l'eau du robinet en cuve on additionne 20 mg/l de thiosulfate de sodium (TSS (Merck 106516)), soit 1 g dans 50 l;

L'échantillon est dopé avec les oocystes de *C*.*parvum* ;

La filtration est faite selon le mode voie externe ;

Après vidange partielle du carter et agitation du module pour récupérer tous les oocystes à la surface externe des fibres grâce au liquide laissé dans le carter, on récupère le concentrat par gravité, puis en injectant de l'air comprimé (0.5 b) dans le carter.

La numération du stock d'oocystes et du concentrat est faite par IMS-IFA.

### Filtration :

### Déroulement :

50 litres d'eau du robinet neutralisée au TSS sont filtrés et après vidange on récupère un concentrat qui constituera un Blanc-(B) ; Cette filtration est suivie d'un rétrolavage pour nettoyer la membrane ;

50 litres d'eau du robinet sont ensuite neutralisés avec le TSS puis dopés avec une dilution I10 du stock d'oocystes (soit environ 10 oocystes).

Après filtration on vidange et on récupère un concentrat (C10) ;

Cette filtration est suivie d'un rétrolavage avec au moins 10 litres d'eau.

Puis la même procédure est répétée en dopant I100 (environ 100 oocystes), on obtient le concentrat C100 puis en dopant 11000 (environ 1000 oocystes) pour obtenir le concentrat C1000 ;

### Analyse :

- numération par IMS/IFA de I10, I100, I1000, B, C10, C100 et C1000.

### Résultats :

| inoculum | | concentrats | | rendement |
|---|---|---|---|---|
| - | - | B | 0 | - |
| I10 | 4 | C10 | 15 | ≈100% |
| I100 | 43 | C100 | 57 | ≈100% |
| I1000 | 335 | C1000 | 236 | 70% |

Les résultats pour C10 et C100 qui sont supérieurs à la valeur mesurée pour l'inoculum ne doivent pas surprendre: l'échantillonnage pour compter les oocystes au microscope implique nécessairement des fluctuations comme on le constate quand on multiplie les comptages d'un même échantillon. Ce phénomène est d'autant plus marqué que les oocystes sont rares.

Ainsi, pour C10 et C100, il faut considérer que l'ordre de grandeur est constant après filtration.

Pour C1000, il est possible de parler de rendement.

### Interprétation des résultats :

La méthode de filtration par voie externe a une sensibilité de 10 oocystes ou moins.

Pour un inoculum de 1000 oocystes, le rendement obtenu est de 70% sans optimisation.

### EXEMPLE 3 :

Protocole et méthode employés identiques à ceux utilisés dans l'exemple 1 à l'exception des moyens de filtration.

La filtration est effectuée en utilisant un moyen de filtration comportant 0,6 m² de surface filtrante en fibres creuses d'acétate de cellulose

L'échantillon est constitué de 50 litres d'eau de réseau dopés avec le bactériophage MS2, il est introduit par voie interne (A).

La récupération du concentrat est effectuée par gravité et sous pression.

Un seul Blanc (B₁) est produit et le dopage est fait successivement avec 1, 10, 100 PFU.

L'analyse est faite par la technique des plages de lyse comme dans l'exemple 1.

Une deuxième étape de filtration des concentrats issus de la filtration précédente est effectuée selon le protocole décrit ci-après.

Le concentrat blanc (B₁) et chacun des concentrats de phages (C₁1, C₁10, C₁100), soit environ 230 ml chacun, sont répartis en trois fractions égales (70-80 ml) pour une deuxième concentration en trois modules commerciaux Centricon-Plus 80 (Millipore réf. UFC5LGC08).

Les trois concentrats C₂ ainsi obtenus (< 1 ml) pour chaque dopage sont mélangés et analysés en titration quantitative par plages de lyse. On procède de même pour les trois concentrats blanc (B₂) issus de la concentration de B₁.

### Résultats :

- titration de l'inoculum : I100 = 144 PFU (moyenne globale comme dans Exemple 1 ) ;
- titration des concentrats obtenus après la deuxième filtration :

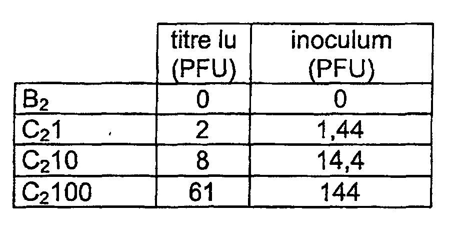

C₂1, par exemple, désigne le mélange des trois concentrats obtenus à l'issue de la deuxième étape de concentration de l'échantillon initial dopé avec 1 PFU.

### Interprétation des résultats :

Sur l'ensemble des deux étapes de concentration, en réduisant le volume de l'échantillon de 50 litres à moins de trois ml, la sensibilité est proche de 1 PFU.

Il demeure bien entendu que la présente invention n'est pas limitée aux exemples de réalisation décrits et/ou représentés, mais qu'elle en englobe toutes.les variantes qui entrent dans le cadre de la portée des revendications annexées (en particulier en ce qui concerne la nature et les caractéristiques des membranes utilisées pour la concentration des échantillons)."

### EXEMPLE 4 :

### Matériel et méthode :

Utilisation d'un moyen de filtration comportant 0,12 m² de surface filtrante en fibres creuses d'acétate de cellulose pour une première phase de filtration et d'un consommable Vivacell70 (Sartorius Vivasciences ref. VS6002) pour une seconde phase de filtration.

L'échantillon est constitué de 30 litres d'eau de réseau dopés simultanément avec 300 oocystes du parasite *Cryptosporidium parvum* et 300 CFU de la bactérie *Escherichia coli.*

### Concentration :

Après prélèvement de l'eau du robinet en cuve on ajoute 20 mg/l de thiosulfate de sodium (Merck 106516), soit 0,6 g dans 30 l;

L'échantillon est dopé avec les oocystes de *C.parvum* et les cellules d'*E*.*coli* ;

### Phase 1 : de 30 litres à 70 ml

La filtration est faite selon le mode voie externe.

Après vidange partielle du carter et agitation du module, on récupère le retentat contenant les microorganismes en injectant de l'air comprimé (0,5 b) dans le carter, soit environ 35 ml.

On réintroduit de l'eau de réseau neutralisée non dopée dans le carter (environ 35 ml) à travers les fibres par injection dans le compartiment interne fermé, puis on agite et on collecte comme précédemment.

L'assemblage des deux prélèvements, soit 70 ml, constitue le retentat R1.

### Phase 2: de 70 ml à 300 microlitres

Le rétentat R1 est concentré dans le consommable Vivacell70 comme indiqué par le fournisseur, en appliquant une force centrifuge de 1000 G pendant 10 min à 25°C. Pour améliorer la récupération, 1 ml de filtrat est appliqué sur le concentrat dans la chambre de filtration puis l'élément filtrant est "vortexé" brièvement à grande vitesse. Une nouvelle centrifugation dans les mêmes conditions que précédemment permet de reconcentrer pour atteindre au plus 300 microlitres. On obtient ainsi le rétentat final R2.

Trois rétentats R2 sont produits en parallèle pour tripler les résultats.

### Analyses :

### Recherche de C. parvum :

La numération du stock d'oocystes et d'une fraction du rétentat est faite par IMS ("immuno -magnetic separation", kit Dynabeads anti-Cryptosporidium de Dynal, ref. 730.01) puis IFA ("immuno fluorescence assay"), (voir Method 1622).

Une fraction du rétentat est analysée par IMS (voir ci-dessus) puis PCR selon le protocole suivant:
- reprendre le complexe billes/oocystes issu de l'IMS dans 10 µl d'eau ultra pure stérile + 10µl de chelex 100 à 50% dans l'eau;
- faire 5 chocs thermiques : 2 min à 95°C / 2 min à -80°C;
- centrifuger pour culotter la condensation;
- ajouter 30 ul de mix d'amplification (tampon PCR, MgCl2: 1,5 mM, Albumine de Serum Bovin: 10 pg/µl, dNTP: 200 pmol/µl, Taq DNA polymerase: 0,5 U, amorces BB-3 et BB-4: 1pmol/µl de chaque, eau ultrapure qsp 50 µl).

Les amorces BB-3 et BB-4 sont décrites dans Balatbat A.B. et al. J. Clin. Microbiol. 1996, 34, 1769-1772.
- appliquer le programme de PCR suivant: 5 min à 94°C, 40 cycles comprenant: [30 sec à 94°C, 45 sec à 60°C, 1 min à 72°C], puis 5 min à 72°C.
- migrer 10 µl de réaction de PCR sur un gel d'électrophorèse à 2% d'agarose en présence de bromure d'éthidium.

### Recherche d'E.coli :

La numération de la culture d'*E*.*coli* et d'une fraction du rétentat est réalisée avec la technique dite "Colilert 18h" selon les recommandations du fournisseur IDEXX. Cette méthode est basée sur la détection de l'activité béta-glucuronidase d'*Escherichia coli*.

Une fraction du rétentat est analysée par nested PCR appliquée au gène *uidA* codant pour la béta-glucuronidase selon le protocole suivant:
- filtrer sur membrane (Millipore, diamètre 13 mm, porosité 0,2 µm Durapore GVWP013 00) de la fraction de concentrat;
- introduire la membrane dans un tube PCR 0,2ml;
- lyser les cellules par chocs thermiques (6 cycles 85°C 1 min / glace 1 min)
- première amplification dans le tube de lyse avec amorces U270 et L1054 U270: 5'-ggT CAC TCA TTA Cgg CAA Ag- 3' L1054: 5'-TTA AAg CCg ACA gCA gCA gT- 3'
   Mix d'amplification (150µl) mis dans le tube PCR contenant la membrane: Tampon PCR, MgCl₂: 1,5 mM, dNTP: 200 pmol/µl, Taq DNA polymerase: 1,5 U, amorces: 1pmol/µl de chaque, eau ultrapure qsp 150 µl.
   Programme de PCR: 10 min à 95°C, 30 cycles comprenant: [1 min à 94°C, 1 min à 60°C], puis maintien à 4°C.
- diluer 100 fois les amplicons obtenus avec de l'eau ultrapure et utiliser 1 µl de cette dilution comme substrat de la deuxième amplification (dite "nested PCR") avec les amorces Val 754 et Val 900: Val 754: 5'-AAA ACg gCA AgA AAA AgC Ag- 3' Val 900: 5'-Acg CgT ggT TAC AgT CTT gCg- 3'
   Mix d'amplification (50µl) mis dans un nouveau tube: Tampon PCR, MgCl₂: 1,5 mM, dNTP: 200 pmol/µl, Taq DNA polymerase: 0,5 U, amorces: 1pmol/µl de chaque, eau ultrapure qsp 50 µl.
   Programme de PCR: identique au précédent.
- migrer 10 µl de réaction de PCR sur un gel d'électrophorèse à 2% d'agarose en présence de bromure d'éthidium.

### Traitement du rétentat R2 :

Le rétentat est divisé en trois fractions de 100 microlitres chacune: R2a, R2b, R2c.

La fraction R2a est redivisée en deux: 50 µl sont traités en "Colilert 18h" (numération *E*.*coli*), 50 µl en IMS-IFA (numération *C*.*parvum*).

La fraction R2b est analysée en nested-PCR uidA (détection moléculaire d'*E*.*coli*), mais au préalable elle est séparée en deux: 50 µl sont analysés directement, 50 µl sont additionnés d'ADN génomique d'*E*.*coli* (test d'inhibition).

La fraction R2c est analysée en IMS-PCR (détection moléculaire de *C*.*parvum*), mais au préalable elle est séparée en deux: 50 µl sont analysés directement, 50 µl sont additionnés d'ADN génomique de *C.parvum* (test d'inhibition).

Les PCR comportent chacune un témoin négatif (pas d'ADN cible, ni de rétentat) et un témoin positif (avec ADN cible mais pas de rétentat).

Tous les amplicons sont visualisés sur gel d'agarose par électrophorèse en présence de bromure d'éthidium.

### Résultats :

Chaque point est reproduit trois fois et les résultats répétés sont séparés par le signe "/" dans le tableau ci-dessous.

| | numération théorique de la fraction analysée | résultat (numération ou bande sur gel) |
|---|---|---|
| numération *E.coli* (Colilert) | 50 | 0/1/2 |
| détection nested PCR uidA *E.coli* | 50 | + / + / + |
| test inhibition PCR uidA | 50 | + / + / + |
| témoin positif PCR uidA | 50 | + |
| témoin négatif PCR uidA | 50 | - |
| numération *C.parvum* (IMS-IFA) | 50 | 1/11/9 |
| détection IMS-PCR *C.parvum* | 50 | + / + / + |
| test inhibition IMS-PCR *C.parvum* | 50 | +/+/+ |
| témoin positif IMS-PCR *C.parvum* | 50 | + |
| témoin négatif IMS-PCR *C.parvum* | 50 | - |

### Interprétation des résultats :

Le faible nombre de cellules *E*.*coli* détectées par Colilert dans le concentrat s'explique par le stress, voire la mortalité, subis par les bactéries pendant la concentration car ce test est basé sur la détection d'une activité enzymatique.

La PCR uidA est positive pour les trois répétitions, ce qui indique une sensibilité reproductible de l'ensemble du procédé de 50 bactéries dans 5 litres, soit 5 bactéries par litre.

Logiquement, l'addition d'ADN bactérien dans une fraction comme témoin d'inhibition donne aussi des réponses positives.

Pour *C*.*parvum*, la méthode phénotypique (IMS-IFA) donnent des numérations significativement plus faibles que la valeur théorique attendue. Là encore, il est probable qu'une partie des oocystes soient altérés pendant la concentration plutôt que retenus dans le dispositif de filtration car d'autres expériences basées sur la biologie moléculaire indiquent une récupération reproductible de 1 oocyste dans 10 litres (données non communiquées dans ce brevet).

L'IMS-PCR est d'ailleurs positive pour les trois répétitions, ce qui donne une sensibilité minimale de 50 oocystes dans 5 litres, soit 5 oocystes par litre.

### Conclusion :

Cet exemple montre l'efficacité du dispositif de concentration en deux étapes pour la détection, dans un même échantillon d'eau dopée, d'une bactérie et d'un parasite par des méthodes de biologie moléculaire.

## Revendications

1. Utilisation d'un moyen de filtration comportant un carter (2) dans lequel sont disposées parallèlement les unes aux autres une multiplicité de fibres creuses (3), dont la paroi poreuse (3a) de chacune détermine une lumière (4), en sorte que ledit moyen comporte une voie interne (A) constituée par la multiplicité des lumières (4) en parallèle desdites fibres creuses respectivement, et une voie externe (B) correspondant au volume disponible entre les fibres et le carter, pour le traitement d'un échantillon liquide de départ de volume important comportant à l'état dilué une multiplicité de microorganismes de tailles différentes, pour obtenir un échantillon liquide final de faible volume, comportant à l'état concentré la même composition relative en microorganismes, utilisation selon laquelle, pendant au moins une étape de concentration :
a. on dispose d'un seul moyen de filtration
b. ledit moyen est utilisé en mode frontal,
c. le seuil de coupure de la paroi poreuse (3a) des fibres est déterminé en sorte d'arrêter le micro-organisme de l'échantillon de départ de plus petite taille, et de manière discontinue :
d. pendant une phase de filtration, on introduit la totalité de l'échantillon liquide de départ par la voie interne (A) du moyen de filtration, on évacue un filtrat par la voie externe (B) du moyen de filtration, et on accumule un concentrat dans ladite voie d'introduction de l'échantillon liquide,
e. pendant une phase de récupération, après avoir fait circuler la totalité de l'échantillon liquide de départ, on arrête la circulation de ce dernier dans le moyen (1) de filtration de ladite voie à ladite autre voie, et on obtient l'échantillon liquide de sortie, c'est-à-dire le concentrat,
f. pendant une phase de rétrolavage, on fait circuler un flux liquide pouvant être exempt de microorganismes, de ladite voie à ladite autre voie.

2. Utilisation d'un moyen de filtration comportant un carter (2) dans lequel sont disposées parallèlement les unes aux autres une multiplicité de fibres creuses (3), dont la paroi poreuse (3a) de chacune détermine une lumière (4), en sorte que ledit moyen comporte une voie interne (A) constituée par la multiplicité des lumières (4) en parallèle desdites fibres creuses respectivement, et une voie externe (B) correspondant au volume disponible entre les fibres et le carter, pour le traitement d'un échantillon liquide de départ de volume important comportant à l'état dilué une multiplicite de microorganismes de tailles différentes, pour obtenir un échantillon liquide final de faible volume, comportant à l'état concentré la même composition relative en microorganismes, utilisation selon laquelle, pendant au moins une étape de concentration :
a. on dispose d'un seul moyen de filtration
b. ledit moyen est utilisé en mode frontal,
c. le seuil de coupure de la paroi poreuse (3a) des fibres est déterminé en sorte d'arrêter le micro-organisme de l'échantillon de départ de plus petite taille, et de manière discontinue :
d. pendant une phase de filtration, on introduit la totalité de l'échantillon liquide de départ par la voie externe (B) du moyen de filtration, on évacue un filtrat par la voie linterne (A) du moyen de filtration, et on accumule un concentrat dans ladite voie d'introduction de l'échantillon liquide,
e. pendant une phase de récupération, après avoir fait circuler la totalité de l'échantillon liquide de départ, on arrête la circulation de ce dernier dans le moyen (1) de filtration de ladite voie à ladite autre voie, et on obtient l'échantillon liquide de sortie, c'est-à-dire le concentrat

3. Utilisation selon la revendication 1 **caractérisée en ce que**, entre la phase de filtration (d) et la phase de récupération (e), la voie externe est vidée et éventuellement mise sous pression d'air.

4. Utilisation selon la revendication 1, **caractérisée en ce que** la phase de récupération (e) est effectuée par écoulement par gravité du concentrat, et/ou circulation d'air sous pression dans la voie interne.

5. Utilisation selon la revendication 1, **caractérisée en ce que** lors de la phase de filtration (d), la circulation de réchantillon liquide de départ dans la vole interne est effectuée par refoulement à l'entrée de la voie interne et/ou aspiration à la sortie de ladite voie interne.

6. Utilisation selon la revendication 2, **caractérisée en ce que**, pendant une phase de rétrolavage, on fait circuler un flux liquide pouvant être exempt de microorganismes, de ladite voie à ladite autre voie.

7. Utilisation selon l'une quelconque des revendications 2 ou 6, **caractérisée en ce que** entre la phase de filtration (d) et la phase de récupération (e), la voie externe est partiellement ou totalement vidée.

8. Utilisation selon l'une quelconque des revendications 2, 6 ou 7, **caractérisée en ce que** l'échantillon liquide final est obtenu par élution du concentrat dans la voie externe.

9. Utilisation selon l'une quelconque des revendications précédentes, **caractérisée en ce que** la phase de filtration (d) est effectuée sans préfiltration de l'échantillon liquide de départ.

10. Utilisation salon l'une quelconque des revendications précédentes, **caractérisée en ce que** le moyen de filtration est un moyen d'ultrafiltration.

11. Utilisation selon l'une quelconque des revendications précédentes, **caractérisée en ce que** les fibres du moyen de filtration sont constituées d'une membrane organique (par exemple en acétate de cellulose, éthylcellulose, polyether sulfone, ou polyacrylonitrile) ou d'une membrane minérale par exemple une céramique.

12. Utilisation selon l'une quelconque des revendications précédentes, **caractérisée en ce que** les fibres creuses du moyen de filtration sont noyées à leurs deux extrémités respectivement dans une résine pour éviter tout passage de contaminant entre la voie interne (A) et la voie externe (B), et entre le moyen de filtration et l'extérieur.

13. Utilisation selon l'une quelconque des revendications précédentes, **caractérisée en ce que** le moyen de filtration est dimensionné et la phase de filtration est effectuée pendant un temps déterminé en sorte que le rapport entre le volume de l'échantillon de départ et le volume de l'échantillon final est au moins égal à 50, et préférentiellement compris entre 150 et 500.

14. Utilisation selon l'une quelconque des revendications précédentes, **caractérisée en ce qu'**on effectue au moins deux étapes successives de concentration d'un échantillon liquide, ces deux étapes étant effectuées par l'utilisation d'un moyen de filtration selon la revendication 1, le concentrat obtenu à l'issue de la première étape de concentration, constituant l'échantillon liquide de départ de la deuxième étape de concentration.

15. Utilisation selon l'une quelconque des revendications 1 à 13 **caractérisée en ce qu'**on effectue au moins deux traitements successifs d'un échantillon liquide, et au moins un de ces traitements étant une étape de concentration selon l'une quelconque des revendications 1 ou 2, le concentrat obtenu à l'issue du premier traitement, constituant l'échantillon liquide de départ du deuxième traitement.

16. Utilisation selon la revendication 14 ou 15 **caractérisée en ce que** les moyens de filtration sont dimensionnés et les traitements sont effectués pendant un temps déterminé, en sorte que le rapport entre le volume de l'échantillon de départ et le volume de l'échantillon final est au moins égal à 5 000 et préfèrentiellement compris entre 15 000 et 100000.

## Patentansprüche

1. Verwendung einer Filtrationsvorrichtung, umfassend ein Gehäuse (2), in dem parallel zueinander eine Mehrzahl Hohlfasern (3) angeordnet ist, deren jeweilige poröse Wand (3a) ein Lumen (4) derart festlegt, dass die Vorrichtung einen internen Weg (A), der aus der Mehrzahl von Lumina (4) jeweils parallel zu den Hohlfasern besteht, und einen externen Weg (B) aufweist, der dem zwischen den Fasern und dem Gehäuse verfügbaren Volumen entspricht, zur Behandlung einer flüssigen Ausgangsprobe mit erheblichem Volumen, die in verdünntem Zustand eine Mehrzahl Mikroorganismen unterschiedlicher Größen umfasst, um eine flüssige Endprobe mit kleinem Volumen zu erhalten, die in konzentriertem Zustand die gleiche jeweilige Zusammensetzung an Mikroorganismen umfasst, wobei gemäß der Verwendung während mindestens eines Schritts der Einengung:
a. eine einzige Filtrationsvorrichtung vorgesehen ist
b. die Vorrichtung im frontalen Modus verwendet wird,
c. die Ausschlussgrenze der porösen Wand (3a) der Fasern derart festgelegt wird, dass der kleinste Mikroorganismus der Ausgangsprobe zurückgehalten wird, und auf diskontinuierliche Weise:
d. während einer Phase der Filtration die Gesamtheit der flüssigen Ausgangsprobe auf dem internen Weg (A) der Filtrationsvorrichtung eingebracht wird, ein Filtrat auf dem externen Weg (B) der Filtrationsvorrichtung entnommen wird und ein Konzentrat in dem Weg, auf dem die flüssige Probe eingebracht wurde, akkumuliert wird,
e. während einer Phase der Rückgewinnung, nachdem man die gesamte flüssige Ausgangsprobe umgeführt hat, die Umführung der Letzteren in der Vorrichtung (1) zur Filtration von dem einen Weg zu dem anderen Weg gestoppt wird und eine flüssige Endprobe, d.h. das Konzentrat, erhalten wird,
f. während einer Phase des Rückspülens ein flüssiger Strom, der frei von Mikroorganismen sein kann, von dem einen Weg zu dem anderen Weg umgeführt wird.

2. Verwendung einer Filtrationsvorrichtung, umfassend ein Gehäuse (2), in dem parallel zueinander eine Mehrzahl Hohlfasern (3) angeordnet ist, deren jeweilige poröse Wand (3a) ein Lumen (4) derart festlegt, dass die Vorrichtung einen internen Weg (A), der aus der Mehrzahl von Lumina (4) jeweils parallel zu den Hohlfasern besteht, und einen externen Weg (B) aufweist, der dem zwischen den Fasern und dem Gehäuse verfügbaren Volumen entspricht, zur Behandlung einer flüssigen Ausgangsprobe mit erheblichem Volumen, die in verdünntem Zustand eine Mehrzahl Mikroorganismen unterschiedlicher Größen umfasst, um eine flüssige Endprobe mit kleinem Volumen zu erhalten, die in konzentriertem Zustand die gleiche jeweilige Zusammensetzung an Mikroorganismen umfasst, wobei gemäß der Verwendung während mindestens eines Schritts der Einengung:
a. eine einzige Filtrationsvorrichtung vorgesehen ist
b. die Vorrichtung im frontalen Modus verwendet wird,
c. die Ausschlussgrenze der porösen Wand (3a) der Fasern derart festgelegt wird, dass der kleinste Mikroorganismus der Ausgangsprobe zurückgehalten wird, und auf diskontinuierliche Weise:
d. während einer Phase der Filtration die Gesamtheit der flüssigen Ausgangsprobe auf dem externen Weg (B) der Filtrationsvorrichtung eingebracht wird, ein Filtrat auf dem internen Weg (A) der Filtrationsvorrichtung entnommen wird und ein Konzentrat in dem Weg, auf dem die flüssige Probe eingebracht wurde, akkumuliert wird,
e. während einer Phase der Rückgewinnung, nachdem man die gesamte flüssige Ausgangsprobe umgeführt hat, die Umführung der Letzteren in der Vorrichtung (1) zur Filtration von dem einen Weg zu dem anderen Weg gestoppt wird und eine flüssige Endprobe, d.h. das Konzentrat, erhalten wird.

3. Verwendung nach Anspruch 1, **dadurch gekennzeichnet, dass** zwischen der Phase der Filtration (d) und der Phase der Rückgewinnung (e) der externe Weg evakuiert und gegebenenfalls unter Luftdruck gesetzt wird.

4. Verfahren nach Anspruch 1, **dadurch gekennzeichnet, dass** die Phase der Rückgewinnung (e) durch Ablaufenlassen des Konzentrats mittels Schwerkraft und/oder Umführen von Luft unter Druck im internen Weg durchgeführt wird.

5. Verwendung nach Anspruch 1, **dadurch gekennzeichnet, dass** während der Phase der Filtration (d) die Umführung der flüssigen Ausgangsprobe im internen Weg durch Anstauen am Eingang des internen Wegs und/oder Ansaugen am Ausgang des internen Wegs durchgeführt wird.

6. Verwendung nach Anspruch 2, **dadurch gekennzeichnet, dass** während einer Phase des Rückspülens ein flüssiger Strom, der frei von Mikroorganismen sein kann, von dem einen Weg zu dem anderen Weg umgeführt wird.

7. Verwendung nach Anspruch 2 oder 6, **dadurch gekennzeichnet, dass** zwischen der Phase der Filtration (d) und der Phase der Rückgewinnung (e) der externe Weg teilweise oder völlig evakuiert wird.

8. Verwendung nach einem der Ansprüche 2, 6 oder 7, **dadurch gekennzeichnet, dass** die flüssige Endprobe durch Elution des Konzentrats im externen Weg erhalten wird.

9. Verwendung nach einem der vorstehenden Ansprüche, **dadurch gekennzeichnet, dass** die Phase der Filtration (d) ohne Vorfiltration der flüssigen Ausgangsprobe durchgeführt wird.

10. Verwendung nach einem der vorstehenden Ansprüche, **dadurch gekennzeichnet, dass** die Filtrationsvorrichtung eine Ultrafiltrationsvorrichtung ist.

11. Verwendung nach einem der vorstehenden Ansprüche, **dadurch gekennzeichnet, dass** die Fasern der Filtrationsvorrichtung aus einer organischen Membran (zum Beispiel Celluloseacetat, Ethylcellulose, Polyethersulfon oder Polyacrylnitril) oder einer anorganischen Membran, zum Beispiel einer Keramik, bestehen.

12. Verwendung nach einem der vorstehenden Ansprüche, **dadurch gekennzeichnet, dass** die Hohlfasern der Filtrationsvorrichtung an ihren beiden Enden jeweils in einem Harz eingebettet sind, um jeglichen Durchtritt von Verunreinigung zwischen dem internen Weg (A) und dem externen Weg (B) und zwischen der Filtrationsvorrichtung und der Umgebung zu verhindern.

13. Verwendung nach einem der vorstehenden Ansprüche, **dadurch gekennzeichnet, dass** die Filtrationsvorrichtung derart dimensioniert ist und dass die Filtrationsphase während einer bestimmten Zeit derart durchgeführt wird, dass das Verhältnis zwischen dem Volumen der Ausgangsprobe und dem Volumen der Endprobe mindestens gleich 50 ist und bevorzugt zwischen 150 und 500 beträgt.

14. Verwendung nach einem der vorstehenden Ansprüche, **dadurch gekennzeichnet, dass** mindestens zwei aufeinander folgende Schritte der Einengung einer flüssigen Probe durchgeführt werden, wobei die zwei Schritte mittels Verwendung einer Filtrationsvorrichtung nach Anspruch 1 durchgeführt werden, wobei das am Ende des ersten Einengungsschritts erhaltene Konzentrat die flüssige Ausgangsprobe des zweiten Einengungsschritts bildet.

15. Verwendung nach einem der Ansprüche 1 bis 13, **dadurch gekennzeichnet, dass** mindestens zwei aufeinander folgende Behandlungen einer flüssigen Probe durchgeführt werden, und wobei mindestens eine der Behandlungen ein Einengungsschritt nach Anspruch 1 oder 2 ist, wobei das am Ende der ersten Behandlung erhaltene Konzentrat die flüssige Ausgangsprobe der zweiten Behandlung bildet.

16. Verwendung nach Anspruch 14 oder 15, **dadurch gekennzeichnet, dass** die Filtrationsvorrichtungen derart dimensioniert sind und dass die Behandlungen während einer bestimmten Zeit derart durchgeführt werden, dass das Verhältnis zwischen dem Volumen der Ausgangsprobe und dem Volumen der Endprobe mindestens gleich 5000 ist und bevorzugt zwischen 15 000 und 100 000 beträgt.

## Claims

1. Use of a filtration means comprising a casing (2) in which a multiplicity of hollow fibres (3) are arranged mutually parallel, the porous parts (3a) of each of which fibres defines a slot (4) so that the said means comprises an internal channel (A) consisting of the multiplicity of respective parallel slots (4) of the said hollow fibres, and an external channel (B) corresponding to the volume available between the fibres and the casing, for the treatment of an initial liquid sample with a large volume comprising a multiplicity of microorganisms of different sizes in the dilute state, in order to obtain a final liquid sample with a small volume comprising the same relative composition of microorganisms in the concentrated state, according to which use, during at least one concentration step:
a. a single filtration means is used,
b. the said means is used in a frontal mode,
c. the cutoff threshold of the porous parts (3a) of the fibres is defined so as to retain the smallest microorganisms in the initial sample, and, discontinuously:
d. during a filtration phase, all of the initial liquid sample is introduced through the internal channel (A) of the filtration means, a filtrate is discharged through the external channel (B) of the filtration means, and a concentrate is accumulated in the said channel for introduction of the liquid sample,
e. during a recovery phase, after all of the initial liquid volume has been circulated, its circulation in the filtration means (1) from the said channel to the said other channel is stopped, and the output liquid sample is obtained, that is to say the concentrate,
f. during a back-washing stage, a liquid flow which may be free of microorganisms is circulated from the said channel to the said other channel.

2. Use of a filtration means comprising a casing (2) in which a multiplicity of hollow fibres (3) are arranged mutually parallel, the porous parts (3a) of each of which fibres defines a slot (4) so that the said means comprises an internal channel (A) consisting of the multiplicity of respective parallel slots (4) of the said hollow fibres, and an external channel (B) corresponding to the volume available between the fibres and the casing, for the treatment of an initial liquid sample with a large volume comprising a multiplicity of microorganisms of different sizes in the dilute state, in order to obtain a final liquid sample with a small volume comprising the same relative composition of microorganisms in the concentrated state, according to which use, during at least one concentration step:
a. a single filtration means is used,
b. the said means is used in a frontal mode,
c. the cutoff threshold of the porous parts (3a) of the fibres is defined so as to retain the smallest microorganisms in the initial sample, and, discontinuously:
d. during a filtration phase, all of the initial liquid sample is introduced through the external channel (B) of the filtration means, a filtrate is discharged through the internal channel (A) of the filtration means, and a concentrate is accumulated in the said channel for introduction of the liquid sample,
e. during a recovery phase, after all of the initial liquid volume has been circulated, its circulation in the filtration means (1) from the said channel to the said other channel is stopped, and the output liquid sample is obtained, that is to say the concentrate.

3. Use according to Claim 1, **characterized in that** the external channel is emptied and optionally placed under air pressure between the filtration phase (d) and the recovery phase (e).

4. Use according to Claim 1, **characterized in that** the recovery phase (e) is carried out by a gravitational flow of the concentrate and/or circulation of air under pressure in the internal channel.

5. Use according to Claim 1, **characterized in that** during the filtration phase (d), the circulation of the initial liquid sample in the internal channel is carried out by pressurization at the inlet of the internal channel and/or suction at the outlet of the said internal channel.

6. Use according to Claim 2, **characterized in that** during a back-washing stage, a liquid flow which may be free of microorganisms is circulated from the said channel to the said other channel.

7. Use according to either one of Claims 2 and 6, **characterized in that** the external channel is partially or completely emptied between the filtration phase (d) and the recovery phase (e).

8. Use according to any one of Claims 2, 6 and 7, **characterized in that** the final liquid sample is obtained by elution of the concentrate in the external channel.

9. Use according to any one of the preceding claims, **characterized in that** the filtration phase (d) is carried out without pre-filtration of the initial liquid sample.

10. Use according to any one of the preceding claims, **characterized in that** the filtration means is an ultrafiltration means.

11. Use according to any one of the preceding claims, **characterized in that** the fibres of the filtration means consist of an organic membrane (for example made of cellulose acetate, ethyl cellulose, polyether sulphone or polyacrylonitrile) or an inorganic membrane, for example a ceramic.

12. Use according to any one of the preceding claims, **characterized in that** the hollow fibres of the filtration means are embedded in a resin at their two respective ends, in order to prevent any transfer of contaminants between the internal channel (A) and the external channel (B) and between the filtration means and the outside.

13. Use according to any one of the preceding claims, **characterized in that** the filtration means is designed, and the filtration phase is carried out for a specific time, so that the ratio between the volume of the initial sample and the volume of the final sample is at least equal to 50, and preferably between 150 and 500.

14. Use according to any one of the preceding claims, **characterized in that** at least two successive steps of concentrating a liquid sample are carried out, these two steps being carried out by using a filtration means according to Claim 1, the concentrate obtained after the first concentration step constituting the initial liquid sample of the second concentration step.

15. Use according to any one of Claims 1 to 13, **characterized in that** at least two successive treatments of a liquid sample are carried out, at least one of these treatments being a concentration step according to either one of Claims 1 and 2, the concentrate obtained after the first treatment constituting the initial liquid sample of the second treatment.

16. Use according to Claim 14 or 15, **characterized in that** the filtration means are designed, and the treatments are carried out for a specific time, so that the ratio between the volume of the initial sample and the volume of the final sample is at least equal to 5000, and preferably between 15,000 and 100,000.
